# FASCICULE DE BREVET EUROPEEN

(11) **EP 3 055 026 B1**
(45) Date de publication et mention de la délivrance du brevet: **07.06.2023**
(21) Numéro de dépôt: 14790228.2
(22) Date de dépôt: 07.10.2014
(51) Int. Cl.: A61N 7/00, A61B 8/08

(54) **PROCEDE DE CARACTERISATION D'UNE LESION ULTRASONORE DE TISSUS ORGANIQUES**
VERFAHREN ZUR CHARAKTERISIERUNG EINER ULTRASCHALLWUNDE IN ORGANISCHEM GEWEBE
METHOD FOR CHARACTERISING AN ULTRASOUND WOUND IN ORGANIC TISSUES

(30) Priorité: 08.10.2013 FR 1359727
(43) Date de publication de la demande: 17.08.2016
(73) Titulaire: EDAP TMS France, 69120 Vaulx-en-Velin (FR); INSERM - Institut National de la Santé et de la Recherche Médicale, 75654 Paris Cedex 13 (FR)
(72) Inventeur: MELODELIMA, David, F-38300 Ruy (FR); VINCENOT, Jérémy, F-69420 Les Haies (FR); BLANC, Emmanuel, F-69370 Saint Didier au Mont d'Or (FR)
(74) Mandataire: Cabinet Beau de Loménie
(86) Numéro de dépôt international: PCT/FR2014/052539
(87) Numéro de publication internationale: WO 2015/052428

(56) Documents cités:
- GARY Y HOU ET AL: "Performance Assessment of HIFU Lesion Detection by Harmonic Motion Imaging for Focused Ultrasound (HMIFU): A 3-D Finite-Element-Based Framework with Experimental Validation", ULTRASOUND IN MEDICINE AND BIOLOGY, NEW YORK, NY, US, vol. 37, no. 12, 6 septembre 2011 (2011-09-06), pages 2013-2027, XP028118768, ISSN: 0301-5629, DOI: 10.1016/J.ULTRASMEDBIO.2011.09.005 [extrait le 2011-09-10]
- SHENG YAN ET AL: "Ultrasound image enhancement for HIFU lesion detection and measurement", 9TH INTERNATIONAL CONFERENCE ON ELECTRONIC MEASUREMENT & INSTRUMENTS, 2009 : ICEMI '09 ; 16 - 19 AUG. 2009, BEIJING, CHINA ; PROCEEDINGS, IEEE, PISCATAWAY, NJ, USA, 16 août 2009 (2009-08-16), pages 4-193, XP031537114, ISBN: 978-1-4244-3863-1
- NOBLE J A ET AL: "Ultrasound image segmentation: a survey", IEEE TRANSACTIONS ON MEDICAL IMAGING, IEEE SERVICE CENTER, PISCATAWAY, NJ, US, vol. 25, no. 8, 1 août 2006 (2006-08-01), pages 987-1010, XP008085509, ISSN: 0278-0062, DOI: 10.1109/TMI.2006.877092
- CHENOT J ET AL: "Intra-operative ultrasound hand-held strain imaging for the visualization of ablations produced in the liver with a toroidal HIFU transducer: first in vivo results;Ultrasound hand-held strain imaging for the visualization of HIFU ablation", PHYSICS IN MEDICINE AND BIOLOGY, INSTITUTE OF PHYSICS PUBLISHING, BRISTOL GB, vol. 55, no. 11, 17 May 2010 (2010-05-17), pages 3131-3144, XP020192440, ISSN: 0031-9155, DOI: 10.1088/0031-9155/55/11/010
- GARY Y HOU ET AL: "Performance Assessment of HIFU Lesion Detection by Harmonic Motion Imaging for Focused Ultrasound (HMIFU): A 3-D Finite-Element-Based Framework with Experimental Validation", ULTRASOUND IN MEDICINE AND BIOLOGY, NEW YORK, NY, US, vol. 37, no. 12, 6 September 2011 (2011-09-06), pages 2013-2027, XP028118768, ISSN: 0301-5629, DOI: 10.1016/J.ULTRASMEDBIO.2011.09.005 [retrieved on 2011-09-10]
- SHENG YAN ET AL: "Ultrasound image enhancement for HIFU lesion detection and measurement", 9TH INTERNATIONAL CONFERENCE ON ELECTRONIC MEASUREMENT & INSTRUMENTS, 2009 : ICEMI '09 ; 16 - 19 AUG. 2009, BEIJING, CHINA ; PROCEEDINGS, IEEE, PISCATAWAY, NJ, USA, 16 August 2009 (2009-08-16), pages 4-193, XP031537114, ISBN: 978-1-4244-3863-1
- NOBLE J A ET AL: "Ultrasound image segmentation: a survey", IEEE TRANSACTIONS ON MEDICAL IMAGING, IEEE, USA, vol. 25, no. 8, 1 August 2006 (2006-08-01) , pages 987-1010, XP008085509, ISSN: 0278-0062, DOI: 10.1109/TMI.2006.877092

## Description

La présente invention concerne le domaine technique des ultrasons focalisés de haute intensité dits HIFU (acronyme anglais pour High Intensity Focused Ultrasound) et elle vise plus précisément la caractérisation d'une lésion de tissus organiques réalisée par l'application d'ondes ultrasonores focalisées.

Dans le traitement des tumeurs cancéreuses solides localisées, l'exérèse chirurgicale reste le plus souvent la meilleure option thérapeutique. En effet, elle permet de retirer le tissu cancéreux et, sous réserve que les marges chirurgicales soient négatives, de minimiser le risque de récidive localisée.

Il existe toutefois deux contraintes à cette approche thérapeutique :
- le geste chirurgical est invasif et peut venir réduire le rapport bénéfice / risque du traitement,
- il convient de s'assurer que les marges chirurgicales sont négatives, c'est-à-dire que la totalité des cellules tumorales ont bien été retirées.

La technologie des ultrasons thérapeutiques HIFU apporte une solution mini invasive intéressante. En effet, le principe consiste à focaliser un faisceau ultrasonore sur la zone tumorale à détruire appelée également zone cible. L'absorption de l'énergie ultrasonore par les tissus biologiques entraîne une importante augmentation de la température qui provoque la nécrose immédiate et irréversible des tissus au point de focalisation du faisceau ultrasonore tout en épargnant les tissus intermédiaires situés entre le transducteur ultrasonore et le point de focalisation.

En revanche, le traitement étant non invasif, le problème du contrôle des marges du traitement est complexe. Lors de l'exérèse, le chirurgien a la possibilité de prélever des tissus biologiques, de les analyser et de vérifier les marges de son geste pour décider de poursuivre ou non l'extension de l'intervention (analyse extemporanée). Ces prélèvements et analyses ne sont plus possibles dans un contexte de traitement non invasif par HIFU.

Plusieurs solutions sont envisageables pour répondre à ce problème. Toutes supposent en premier lieu une imagerie préopératoire ou peropératoire de qualité qui permettra de visualiser précisément la localisation de la zone de tissus à détruire. A ce stade, sont souvent établies des marges thérapeutiques c'est-à-dire une distance minimum autour de la zone cible à laquelle le traitement doit être étendu. Lors du traitement, des dispositifs d'imagerie temps réel permettent d'imager l'organe à traiter, de visualiser la zone cible et de positionner le dispositif thérapeutique en regard de celle-ci. A l'issue du traitement, plusieurs solutions peuvent alors être proposées pour contrôler les marges thérapeutiques selon que la lésion biologique réalisée est visible ou non avec les moyens d'imagerie embarqués dans le dispositif de traitement.

Lorsque la lésion biologique n'est pas visible (traitement par radiothérapie, traitement par ultrasons focalisés sur certains organes tels que la prostate), des outils informatiques (fusion d'images multimodales) sont souvent utilisés pour confirmer que le positionnement du dispositif thérapeutique a bien été réalisé conformément à la planification préopératoire. Mais, malgré l'utilisation de ces outils de « recalage », il convient de pratiquer des marges thérapeutiques suffisamment larges pour avoir la certitude que la totalité de la zone visée a été traitée.

Lorsque la lésion biologique réalisée est visible, l'observation visuelle des marges est possible et l'opérateur vérifie sur différents plans de coupe que la lésion biologique couvre toujours la totalité de la zone cible. Cependant, il convient encore de vérifier que la lésion biologique, telle que visualisée, est bien représentative de la zone de tissu biologique détruite. L'image de la zone biologique détruite peut, par exemple, reposer sur la dé-vascularisation locale des tissus créée par le principe thérapeutique qui se traduira par un hypo signal sur l'imagerie IRM ou ultrasonore. Toutefois, la dé-vascularisation n'est pas absolument représentative de la mort cellulaire et un risque de récidive locale ne peut être totalement écarté.

Sauf à disposer d'un moyen d'imagerie strictement représentatif de la mort cellulaire compatible avec un examen non invasif, le problème du contrôle des marges thérapeutiques lors de traitement mini invasif n'est pas entièrement résolu.

De nouvelles modalités d'imagerie ultrasonore, aussi appelée imagerie échographique, sont aujourd'hui développées pour tenter d'imager des lésions tissulaires non observables en imagerie échographique conventionnelle de type mode B (mode brillance bidimensionnel).

Certaines reposent sur la différence d'élasticité des tissus après leur échauffement par le traitement par ultrasons (imagerie élastographique). Le document « Performance assessment of HIFU Lesion Détection by Harmonie Motion Imaging for Focused Ultrasound (HMIFU) : A 3-D Finite Element-based Framework with experimental Validation, Gary Y Hou et al, Ultrasounds in Medicine and Bioblogy, New York, NY, US, vol.37, n°12, 6 Septembre 2011*"* présente une telle méthode. Ces méthodes sont complexes et nécessitent des systèmes d'imagerie spécifiques et coûteux, qui ne sont pas universellement déployés.

Des algorithmes de traitement d'images spécifiques permettent parfois de rehausser les différences de contraste entre des tissus traités et non traités dans une image échographique conventionnelle de type mode B. Ces méthodes sont complexes et manquent souvent de robustesse pour être appliquées en pratique clinique. Le document « Uitrasound Image Enhancement for HIFU Lesion Détection and Measurement- Sheng Yan et al - 9th International Conférence On Electronic Measurement & Instruments, 2009 - 15-19 Aug 2009, Beijing, China - Proceedings, IEEE, pages 4-193 - ISBN : 978-1-4244-3863-1 - *section III Experimental result on HIFU monitor image* » propose une telle méthode. Si le rehaussement des différences de contraste facilite la différentiation des tissus traités et non traités, ce que l'on appelle segmentation, il est encore nécessaire de recourir à des algorithmes puissants pour permettre une segmentation automatique des contours de la zone traitée. Le document « Ultrasaund Segmentation : A Survey, J. Alison Noble, IEEE Transactions on Medical Imaging Vol 25, n°8, August 2006, pages 987-1010 », propose une revue des récents développements dans le domaine de la segmentation et souligne la difficulté particulière de ce problème dans le domaine de l'imagerie ultrasonore.

Le document par Chenot J. et al. «Intra-operative ultrasound hand-held strain imaging for the visualization of ablations produced in the liver with a toroidal HIFU transducer: first in vivo results» Physics in medicine and biology, vol. 55, no. 11, 17 mai 2010, pages 3131-3144, propose une étude utilisant des images en élastographie pour la visualisation en temps réel des ablations HIFU (ultrasons focalisés de haute intensité) produites dans des tissus animaux par un transducteur toroïdal. Les méthodes de traitement d'image et de segmentation évoquées ci-dessus restent complexes et encore insuffisamment robustes pour être utilisées de manière généralisée. Il apparaît ainsi le besoin de développer un procédé de caractérisation des lésions de tissus organiques traités par des méthodes non invasives ou mini-invasives telles que les traitements HIFU.

L'objet de l'invention vise donc un procédé de caractérisation d'une lésion ultrasonore de tissus organiques, réalisée par l'application d'ultrasons focalisés de haute intensité délivrés par une sonde dont la surface d'émission présente une géométrie torique ou pseudo-cylindrique.

Selon l'invention, le procédé consiste :
- à acquérir après un délai d'au moins deux jours à compter de la fin de l'application des ultrasons, au moins une image de caractérisation des tissus organiques traités,
- à détecter dans l'image la présence d'un liseré de contraste, et
- à partir du liseré de contraste, à déterminer l'extension de la lésion ultrasonore.

Selon des modes de réalisation particuliers, le procédé présente une ou plusieurs des caractéristiques additionnelles suivantes, voire toutes ces caractéristiques :
- détecter, dans l'image de caractérisation, la présence d'un liseré de contraste fermé,
- identifier, dans l'image de caractérisation, la lésion ultrasonore à l'intérieur du liseré de contraste,
- acquérir une image de caractérisation de nature ultrasonore et à identifier à l'intérieur du liseré de contraste de l'image de caractérisation ultrasonore, une zone hyperéchogène et une zone hypoéchogène localisée aux abords de la zone hyperéchogène et du liseré de contraste,
- acquérir une image de référence des tissus organiques avant l'application des ultrasons, pour visualiser la tumeur et à comparer cette image de référence avec l'image de caractérisation afin de contrôler les marges thérapeutiques,
- traiter les images de référence et de caractérisation pour déterminer l'extension de la lésion ultrasonore,
- calculer un rapport entre la surface délimitée par la lésion ultrasonore sur l'image de caractérisation et la surface délimitée par la tumeur sur l'image de référence, pour en déduire un taux de marge d'extension de lésion,
- calculer à partir de plusieurs images de caractérisation et plusieurs images de référence, un rapport entre le volume délimité par la lésion ultrasonore sur les images de caractérisation et le volume délimité par la tumeur sur les images de référence pour en déduire un taux de marge d'extension de la lésion ultrasonore,
- acquérir l'image de caractérisation dans un délai compris dans une gamme allant de six à trente jours et de préférence de l'ordre de huit jours,
- caractériser la lésion de tissus organiques correspondant aux tissus du foie.

Le procédé de caractérisation défini ci-dessus ne contient pas d'étape visant à appliquer des ultrasons focalisés de haute intensité, et donc exclut toute étape de traitement chirurgical ou thérapeutique du corps humain ou animal. Il ne vise qu'à caractériser une lésion qui aurait été provoquée par un tel traitement. Il peut comprendre une ou plusieurs étapes mises en œuvre avant un tel traitement, sans toutefois inclure le traitement.

Par ailleurs, un autre objet de l'invention est de proposer un procédé de traitement et de caractérisation de tissus biologiques dans un corps humain ou animal, ce procédé comportant une étape de traitement incluant une étape d'application d'ultrasons focalisés de haute intensité, délivrés par une sonde dont la surface d'émission présente une géométrie torique ou pseudo-cylindrique, sur des tissus biologiques dans un corps humain ou animal, et incluant le procédé de caractérisation défini ci-dessus.

Diverses autres caractéristiques ressortent de la description faite ci-dessous en référence aux dessins annexés qui montrent, à titre d'exemples non limitatifs, des formes de réalisation de l'objet de l'invention.
La **Figure 1** représente une sonde de thérapie à ultrasons dont la surface d'émission présente une géométrie torique.
La **Figure 2** représente schématiquement l'utilisation d'une telle sonde pour le traitement de tissus organiques.
La **Figure 3** est une image échographique d'une tumeur avant traitement par HIFU.
La **Figure 4** est une image échographique, d'une lésion, acquise à la fin d'une application d'ultrasons.
La **Figure 5** est une image échographique, d'une lésion, acquise après un délai de huit jours à compter de la fin de l'application des ultrasons.

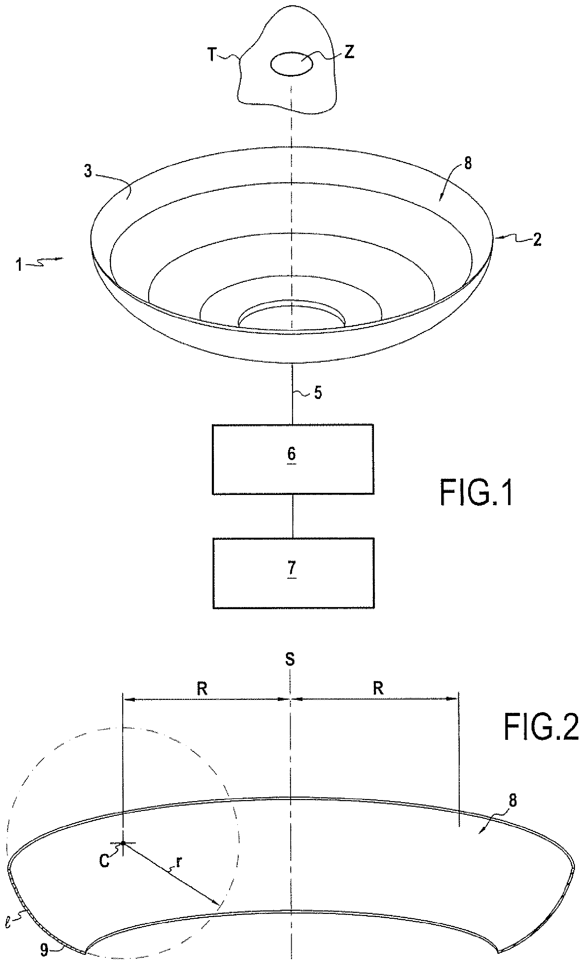

Dans la présente description, l'invention est décrite dans son application à la caractérisation d'une lésion ultrasonore de tissus organiques, réalisée par l'application d'ultrasons focalisés de haute intensité. Ces ultrasons sont délivrés par une sonde de thérapie **1** représentée en **Fig. 1** et dont la surface d'émission présente une géométrie torique. Cette sonde de thérapie **1** est adaptée pour réaliser le traitement de tissus d'un être vivant par l'intermédiaire d'ultrasons focalisés de haute intensité (HIFU). La sonde de thérapie **1** comporte notamment un transducteur **2** comportant un ou plusieurs émetteurs ultrasonores **3** tels que par exemple des éléments piézoélectriques. Ces émetteurs ultrasonores **3** sont reliés par l'intermédiaire de câbles coaxiaux **5** via, un étage amplificateur **6** à un circuit de commande **7** délivrant des signaux pour activer les émetteurs ultrasonores **3.** Le circuit de commande **7** n'est pas décrit plus précisément car sa réalisation fait partie des connaissances techniques de l'homme du métier. Ce circuit de commande **7** comporte ainsi classiquement un générateur de signal piloté qui est relié aux émetteurs ultrasonores par l'intermédiaire de l'étage amplificateur **6.**

Le transducteur **2** présente une face **8** d'émission d'ondes ultrasonores focalisées sur une zone focale **Z.** Comme illustré plus particulièrement à la **Fig. 2****,** cette face d'émission **8** est une surface de révolution engendrée par la rotation autour d'un axe de symétrie **5** d'un segment de courbe concave ou convexe **9,** de longueur **l** et présentant un centre de courbure **C** qui se trouve à une distance **R** de l'axe de symétrie **S,** avec **R** différent de zéro. La surface de révolution **8** est engendrée par un segment d'arc de cercle de longueur **l,** de rayon **r** et avec un centre **c** qui se trouve à une distance **R** de l'axe de symétrie **S,** avec **R** différent de zéro. La géométrie de cette surface de révolution **8** est considérée comme présentant une géométrie torique.

Dans l'exemple illustré à la **Fig. 2****,** le centre de courbure **C** et le segment de courbe **9** sont disposés du même côté de l'axe de symétrie **S.** Il est à noter qu'il peut être prévu que le centre de courbure **C** se trouve situé du côté opposé au segment de courbe **9,** par rapport à l'axe de symétrie **S.** Selon cette variante de réalisation, la face d'émission **8** est considérée comme issue d'une géométrie torique croisée.

Selon l'exemple de réalisation illustré à la **Fig. 2****,** la surface de révolution **8** est engendrée par un segment d'un arc de cercle dont la concavité est tournée vers l'axe de symétrie **S.** Bien entendu, la surface de révolution **8** peut être engendrée par un segment d'une courbe différente d'un arc de cercle. Ainsi, la surface de révolution **8** peut être engendrée par un segment d'une courbe dont la distance **r** entre chaque point du segment de courbe et le centre de courbure **C** présente une variation continue (sans point d'inflexion) tel qu'un segment de courbe elliptique par exemple.

Tel que cela ressort de la description ci-dessus, la face d'émission **8** du transducteur est de géométrie torique. D'une manière générale, la face d'émission **8** du transducteur présente une forme qui est fonction de la géométrie du segment de courbe engendrant la surface émettrice d'ultrasons par rotation autour d'un axe de symétrie, le segment de courbe pouvant présenter des formes diverses. Ainsi, par exemple, la face d'émission **8** peut être engendrée par la translation de deux segments de courbe symétriques, selon une direction perpendiculaire au plan de profil contenant les deux segments de courbe. Chaque segment de courbe est de forme concave et de longueur finie. Selon cette variante de réalisation, la face d'émission **8** présente une géométrie pseudo-cylindrique.

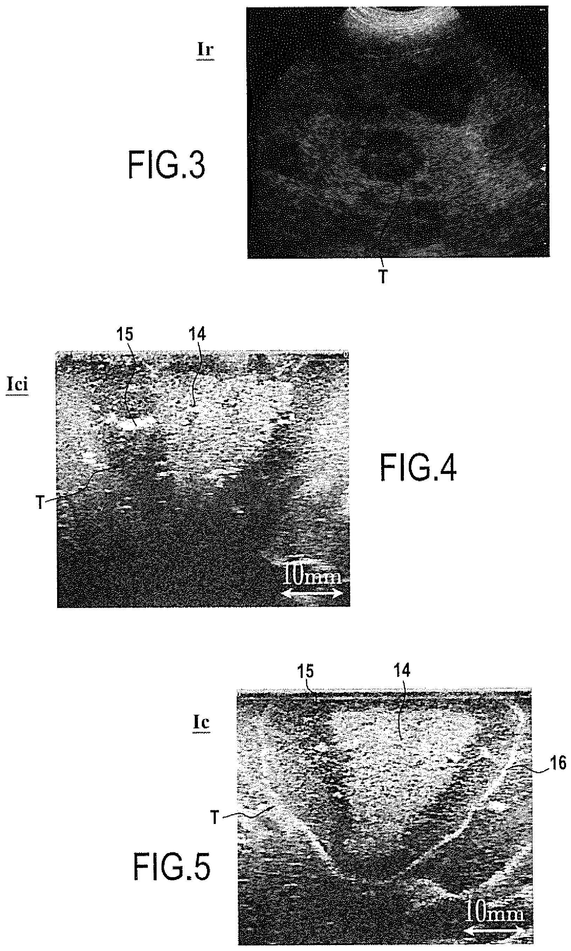

De manière connue, cette sonde ultrasonore **1** est positionnée par abord extracorporel, peropératoire ou endocavitaire, de manière que la zone focale **Z** des ondes ultrasonores ait pour effet de créer une lésion ultrasonore des tissus organiques au niveau de la zone cible ou de traitement **T** correspondant à la tumeur à traiter. La **Fig. 3** est un exemple d'image **Ir** d'une zone cible **T** correspondant à la tumeur à traiter. Cette image dite de référence **Ir** est une image préopératoire ou peropératoire de la zone cible **T** réalisée avant le traitement HIFU.

Classiquement, la zone focale **Z** des ondes ultrasonores est déplacée pour traiter l'ensemble de la zone cible **T,** afin d'obtenir une lésion ultrasonore dans cette zone cible **T** c'est-à-dire une nécrose des tissus dans cette zone. Un tel déplacement est obtenu par le déplacement de la sonde ultrasonore **1** ou par le pilotage électronique des émetteurs ultrasonores **3.** Cette phase d'application des ondes ultrasonores par la sonde **1** ne sera pas décrite plus en détail car elle est bien connue de l'homme du métier et ne fait pas partie de l'objet de l'invention.

Le procédé selon l'invention vise donc à caractériser une telle lésion ultrasonore de tissus organiques dans la zone cible **T,** réalisée par l'application d'ultrasons focalisés de haute intensité délivrés par la sonde dont la surface d'émission présente une géométrie torique ou pseudo-cylindrique. L'invention sera décrite dans la suite de la description dans son application à l'exemple du foie. Nonobstant, elle peut s'appliquer aussi à d'autres tissus organiques du corps humain comme par exemple, le pancréas, les seins, l'utérus, les reins ou le placenta.

La **Fig. 4** représente une image de caractérisation dite initiale **Ici** de la lésion ultrasonore, acquise dès la fin de l'application des ultrasons. Selon l'exemple de réalisation décrit, la **Fig. 4** est une image échographique de la zone cible **T** acquise à l'aide d'un système d'imagerie ultrasonore. Nonobstant, il est bien entendu que l'invention ne se limite pas à des images échographiques mais comprend également les images obtenues par IRM ou par scanner.

Il apparaît sur cette **Fig. 4****,** une zone hyperéchogène **14,** caractéristique de la lésion ultrasonore. En effet, lors de l'échauffement par focalisation des ultrasons, les hépatocytes ou cellules du foie de la zone focale sont détruits. Dans cette zone nécrosée, de nombreuses cavités apparaissent. Chaque cavité est localisée au centre d'un lobule hépatite et coïncide avec la veine centrale. La présence de ces cavités ou espaces tissulaires explique l'aspect hyperéchogène de cette zone de lésion ultrasonore **14.**

Par ailleurs, l'image de caractérisation **Ici** montre également une zone hypoéchogène **15** localisée aux abords de la zone hyperéchogène **14,** et soumise, lors de l'application des ondes ultrasonores, à une élévation de température inférieure à la température de la zone hyperéchogène **14.** Dans cette zone hypoéchogène **15,** le traitement ultrasonore entraine une lyse cellulaire, destruction des organites cytoplasmiques initialement présents dans les hépatocytes. La disparition de ces organites explique l'aspect hypoéchogène de cette zone de lésion **15** entourant la zone nécrosée **14.**

L'objet de l'invention est donc de caractériser la lésion ultrasonore **T** correspondant à la zone hyperéchogène **14** et à la zone hypoéchogène **15.** A cet effet, le procédé selon l'invention consiste tout d'abord à acquérir après un délai d'au moins deux jours à compter de la fin de l'application des ultrasons, au moins une image de caractérisation **Ic** des tissus organiques. Préférentiellement, le délai d'au moins deux jours est compris dans la gamme allant de six à trente jours et typiquement de l'ordre de huit jours. Cette image de caractérisation **Ic,** représentée en **Fig. 5****,** est une image échographique de la zone traitée réalisée par un système d'imagerie ultrasonore mais il clair qu'une telle image de caractérisation peut être obtenue par des systèmes d'imagerie de type différent.

Selon une caractéristique essentielle, le procédé consiste ensuite à détecter sur cette image de caractérisation **Ic,** la présence d'un liseré de contraste **16** en vue de déterminer l'extension de la lésion. Par liséré de contraste, il est compris une ligne plus ou moins continue, de largeur faible et présentant une intensité lumineuse supérieure à celle des tissus environnants correspondant à un hyper signal. Le liseré de contraste **16** apparaît clair dans l'exemple illustré à la **Fig. 5****.** Avantageusement, le procédé consiste à détecter sur cette image, la présence d'un liseré de contraste **16** présentant un contour fermé.

Avantageusement, il apparaît que ce liseré peut aisément être détecté sur une image obtenue par imagerie ultrasonore conventionnelle, c'est-à-dire de type mode B (mode brillance bidimensionnel), largement disponible aujourd'hui et à faible coût, sans nécessiter de recourir à des algorithmes complexes de traitement d'image visant à rehausser les contrastes insuffisants entre les zones traitées et non traitées. En effet, le procédé permet de détecter un liseré de contraste par détection d'une rupture d'impédance acoustique dans les tissus analysés, rupture d'impédance acoustique qui est directement détectée et visible dans l'imagerie ultrasonore conventionnelle de type mode B sans nécessiter de traitement additionnel particulier.

Il doit être compris que lors du traitement HIFU, la chaleur apportée par l'émission ultrasonore entraîne une fragilisation de la membrane plasmique des cellules périphériques. Cette membrane altérée devient alors poreuse. Par effet d'osmose, une forte quantité de calcium pénètre dans le cytoplasme. Si la cellule est toujours fonctionnelle, la régulation de la concentration intracellulaire en calcium est alors prise en charge par les mitochondries. Lorsque la concentration ionique augmente, la mitochondrie convertit le calcium dissous en un précipité solide. Ce phénomène étant permanent, chaque mitochondrie devient alors un germe minéral permettant l'initialisation d'une croissance cristalline. Par la suite, une multitude de grains de calcium sont ainsi formés entraînant la formation du liseré **16.**

Typiquement, sur l'image échographique, le liseré **16** est hyperéchogène. De manière plus précise, le liseré **16** se trouve formé par des précipités d'hydroxyapatite. En effet, sur l'image de caractérisation, apparaissent comme expliqué ci-dessus, de nombreux ilots de matière solide correspondant à des amas de calcium précipités. Entre l'application des ultrasons et la prise de l'image de caractérisation, l'organisme a créé une enveloppe de calcium autour de la lésion proprement dite. La rupture d'impédance acoustique, provoquée lors du passage des ultrasons depuis le tissu hépatique vers le milieu calcifié entraîne la création d'un signal hyperéchogène sur l'image de caractérisation **Ic** qui prend la forme de ce liseré de contraste.

L'apparition de ce liseré **16** au cours du temps après un délai d'au moins deux jours et typiquement d'au moins six jours, permet de contrôler les zones détruites à la suite du traitement ultrasonore. En effet, les hépatocytes situés au-delà du liseré **16,** ne sont pas détruites de manière irréversible et présentent un fonctionnement normal sans précipitation de calcium. Inversement, les hépatocytes situés en-deçà du liseré **16** ont été soumis aux échauffements dus au traitement HIFU et sont caractérisés par une nécrose de coagulation irréversible. Leur fonctionnement étant totalement altéré, les hépatocytes ne précipitent pas de calcium. Les hépatocytes situés dans la zone de transition entre tissus sains et détruits sont seulement fragilisés et conservent une activité suffisante pour précipiter du calcium et former le liseré de contraste. Le liseré **16** permet donc de caractériser l'extension de la lésion ultrasonore **T.**

La formation des zones hyperéchogène **14** et hypoéchogène **15** ainsi que la formation du liseré **16** résultent de la géométrie torique ou pseudo-cylindrique du transducteur ultrasonore **2** utilisé. En comparaison aux technologies actuelles (géométrie sphérique) focalisant les ondes ultrasonores dans de petits volumes juxtaposés les uns aux autres, les géométries toriques ou pseudo-cylindriques permettent de déposer l'énergie ultrasonore de manière massive, dans un volume important créant une vaste zone centrale soumise à une insonification intense dans laquelle se forment des cavités donnant l'aspect hyperéchogène. En périphérie de cette zone, l'insonification est moins intense et provoque une simple lyse cellulaire apparaissant sous la forme d'une image hypoéchogène. Le liseré de contraste **16** se forme à partir des cellules situées dans la fine zone de transition entre les cellules lysées et les cellules saines.

Tel que cela apparaît plus précisément sur la **Fig. 5****,** le liseré **16** entoure la zone hypoéchogène **15** qui elle-même comme expliqué précédemment, entoure la zone hyperéchogène **14.** La zone hypoéchogène **15** et la zone hyperéchogène **14** restent présentes dans cette image de caractérisation **Ic.** L'invention permet avantageusement d'identifier, une fois le liseré **16** détecté dans l'image de caractérisation **Ic,** la lésion ultrasonore **T** à savoir, la zone hyperéchogène **14** et la zone hypoéchogène **15** localisée aux abords de la zone hyperéchogène **14** et du liseré de contraste **16.**

Ainsi, selon l'invention, il suffit d'identifier la présence de ce liseré **16** dans l'image de caractérisation **Ic** pour déterminer l'extension de la lésion. Cette identification de ce liseré **16** pour déterminer l'extension de la lésion ultrasonore peut selon l'invention être exécutée visuellement ou selon un traitement d'image numérique. Typiquement, la détection du liseré **16** consiste par traitement d'images à détecter un liseré fermé ou une boucle fermée, formé d'un ensemble de points.

Selon un exemple préféré de mise en oeuvre de l'invention, le procédé consiste à acquérir une image de référence **Ir** des tissus organiques avant l'application des ultrasons pour visualiser la tumeur comme par exemple dans une image illustrée à la **Fig. 3****.** Le procédé consiste à comparer cette image de référence **Ir** avec l'image de caractérisation **Ic** afin de contrôler les taux de marge et de confirmer la présence de marges négatives.

De plus, la comparaison entre les images de référence **Ir** et de caractérisation **Ic** permet de déduire un taux de marge d'extension de la lésion. Pour cela, il suffit, selon l'invention, de calculer un rapport entre la surface délimitée par la lésion ultrasonore sur l'image de caractérisation **Ic** et la surface délimitée par la tumeur sur l'image de référence **Ir** afin d'en déduire un taux de marge d'extension de lésion ultrasonore. Selon un mode de réalisation avantageux, l'invention consiste à réaliser plusieurs images de référence **Ir** ainsi que plusieurs images de caractérisation **Ic.** Cela permet ainsi, de déterminer d'une part un volume délimité par la lésion ultrasonore sur les images de caractérisation **Ic** et d'autre part le volume délimité par la tumeur sur les images de référence **Ir,** puis d'en déduire un taux de marge d'extension de la lésion ultrasonore en fonction des volumes.

## Revendications

1. **-** Procédé de caractérisation d'une lésion ultrasonore (**T)** de tissus organiques, réalisée par l'application préalable d'ultrasons focalisés de haute intensité délivrés par une sonde (**2**) dont la surface d'émission présente une géométrie torique, ou pseudo-cylindrique, le procédé de caractérisation étant **caractérisé en ce qu'**il comprend les étapes consistant :
- à acquérir après un délai d'au moins deux jours à compter de la fin de l'application des ultrasons, au moins une image à ultrasons de caractérisation (**Ic**) des tissus organiques humains,
- à détecter, selon un traitement d'image numérique, dans l'image de caractérisation (**Ic**), la présence d'une ligne hyperéchogène (**16**) correspondant à des amas de calcium précipités, et
- à partir de la ligne hyperéchogène, à déterminer l'extension de la lésion ultrasonore.

2. - Procédé selon la revendication 1, **caractérisé en ce qu'**il comprend l'étape consistant à détecter, dans l'image de caractérisation (**Ic**), la présence d'un liseré de contraste (**16**) présentant un contour fermé.

3. - Procédé selon la revendication 1 ou 2, **caractérisé en ce qu'**il comprend l'étape consistant à identifier, dans l'image de caractérisation (**Ic**), la lésion ultrasonore à l'intérieur du liseré de contraste (**16**).

4. **-** Procédé selon l'une des revendications 1 à 3, **caractérisé en ce qu'**il comprend l'étape consistant à acquérir une image de caractérisation (**Ic**) de nature ultrasonore et à identifier à l'intérieur du liseré de contraste (**16**) de l'image de caractérisation ultrasonore (**Ic**), une zone hyperéchogène (**14**) et une zone hypoéchogène (**15**) localisée aux abords de la zone hyperéchogène (**14**) et du liseré de contraste (**16**).

5. - Procédé selon l'une des revendications 1 à 4, **caractérisé en ce qu'**il comprend l'étape consistant :
- à utiliser une image de référence (**Ir**) des tissus organiques acquise avant l'application des ultrasons, pour visualiser la tumeur et à comparer cette image de référence (**Ir**) avec l'image de caractérisation afin de contrôler les marges thérapeutiques.

6. - Procédé selon la revendication 5, **caractérisé en ce qu'**il comprend l'étape consistant à traiter les images de référence (**Ir**) et de caractérisation (**Ic**) pour déterminer l'extension de la lésion ultrasonore.

7. - Procédé selon la revendication 5 ou 6, **caractérisé en ce qu'**il comprend l'étape consistant à calculer un rapport entre la surface délimitée par la lésion ultrasonore sur l'image de caractérisation (**Ic**) et la surface délimitée par la tumeur sur l'image de référence (**Ir**), pour en déduire un taux de marge d'extension de lésion.

8. - Procédé selon la revendication 5 ou 6, **caractérisé en ce qu'**il comprend l'étape consistant à calculer à partir de plusieurs images de caractérisation (**Ic**) et plusieurs images de référence (**Ir**), un rapport entre le volume délimité par la lésion ultrasonore sur les images de caractérisation (**Ic**) et le volume délimité par la tumeur sur les images de référence (**Ir**) pour en déduire un taux de marge d'extension de la lésion ultrasonore.

9. - Procédé selon les revendications 1 à 8, **caractérisé en ce que** le délai d'au moins deux jours pour acquérir l'image de caractérisation (**Ic**) est compris dans une gamme allant de six à trente jours et de préférence de l'ordre de huit jours.

10. - Procédé selon les revendications 1 à 9, **caractérisé en ce qu'**il consiste à caractériser la lésion de tissus organiques correspondant aux tissus du foie.

## Patentansprüche

1. Verfahren zur Charakterisierung einer Ultraschall-Läsion (T) von organischen Geweben, durchgeführt durch die vorherige Anwendung von hochintensivem fokussiertem Ultraschall, der von einer Sonde (2) geliefert wird, deren Emissionsfläche eine torische oder pseudo-zylindrische Geometrie aufweist, wobei das Charakterisierungsverfahren **dadurch gekennzeichnet ist, dass** es die Schritte umfasst, welche darin bestehen:
- nach einem Zeitraum von wenigstens zwei Tagen ab dem Ende der Ultraschallanwendung wenigstens ein Ultraschall-Charakterisierungsbild (Ic) der menschlichen organischen Gewebe aufzunehmen,
- in dem Charakterisierungsbild (Ic) gemäß einer digitalen Bildverarbeitung das Vorhandensein einer hyperechogenen Linie (16), welche ausgefällten Kalziumanhäufungen entspricht, zu erkennen, und
- aus der hyperechogenen Linie die Ausdehnung der Ultraschall-Läsion zu bestimmen.

2. Verfahren nach Anspruch 1, **dadurch gekennzeichnet, dass** es den Schritt umfasst, welcher darin besteht, in dem Charakterisierungsbild (Ic) das Vorhandensein eines Kontrastsaums (16), der eine geschlossene Kontur aufweist, zu erkennen.

3. Verfahren nach Anspruch 1 oder 2, **dadurch gekennzeichnet, dass** es den Schritt umfasst, welcher darin besteht, in dem Charakterisierungsbild (Ic) die Ultraschall-Läsion innerhalb des Kontrastsaums (16) zu identifizieren.

4. Verfahren nach einem der Ansprüche 1 bis 3, **dadurch gekennzeichnet, dass** es den Schritt umfasst, welcher darin besteht, ein ultraschallartiges Charakterisierungsbild (Ic) aufzunehmen und innerhalb des Kontrastsaums (16) des Ultraschall-Charakterisierungsbildes (Ic) einen hyperechogenen Bereich (14) sowie einen hyperechogenen Bereich (15),der auf die Umgebungen des hyperechogenen Bereichs (14) und des Kontrastsaums (16) örtlich begrenzt ist, zu identifizieren.

5. Verfahren nach einem der Ansprüche 1 bis 4, **dadurch gekennzeichnet, dass** es den Schritt umfasst, welcher darin besteht:
- ein Referenzbild (Ir) der organischen Gewebe, das vor der Ultraschallanwendung aufgenommen wurde, zu verwenden, um den Tumor sichtbar zu machen, und dieses Referenzbild (Ir) mit dem Charakterisierungsbild zu vergleichen, um die therapeutischen Breiten zu überprüfen.

6. Verfahren nach Anspruch 5, **dadurch gekennzeichnet, dass** es den Schritt umfasst, welcher darin besteht, das Referenzbild (Ir) und das Charakterisierungsbild (Ic) zu verarbeiten, um die Ausdehnung der Ultraschall-Läsion zu bestimmen.

7. Verfahren nach Anspruch 5 oder 6, **dadurch gekennzeichnet, dass** es den Schritt umfasst, welcher darin besteht, ein Verhältnis zwischen der durch die Ultraschall-Läsion begrenzten Fläche in dem Charakterisierungsbild (Ic) und der durch den Tumor begrenzten Fläche in dem Referenzbild (Ir) zu berechnen, um hieraus eine Spanne einer Ausdehnung einer Läsion abzuleiten.

8. Verfahren nach Anspruch 5 oder 6, **dadurch gekennzeichnet, dass** es den Schritt umfasst, welcher darin besteht, anhand von mehreren Charakterisierungsbildern (Ic) und mehreren Referenzbildern (Ir) ein Verhältnis zwischen dem durch die Ultraschall-Läsion begrenzten Volumen in den Charakterisierungsbildern (Ic) und dem durch den Tumor begrenzten Volumen in den Referenzbildern (Ir) zu berechnen, um hieraus eine Spanne einer Ausdehnung der Ultraschall-Läsion abzuleiten.

9. Verfahren nach den Ansprüchen 1 bis 8, **dadurch gekennzeichnet, dass** der Zeitraum von wenigstens zwei Tagen zum Aufnehmen des Charakterisierungsbildes (Ic) in einem Bereich von sechs bis dreißig Tagen und vorzugsweise in der Größenordnung von acht Tagen liegt.

10. Verfahren nach den Ansprüchen 1 bis 9, **dadurch gekennzeichnet, dass** es darin besteht, die Läsion organischer Gewebe zu charakterisieren, die den Geweben der Leber entsprechen.

## Claims

1. A method of characterizing an ultrasound lesion (T) in organic tissues, the lesion being made by applying high intensity focused ultrasound delivered by a probe (2) having its emission surface presenting a shape that is toroidal or pseudo-cylindrical, the method being **characterized in that** it consists in:
· after a period of at least two days from the end of ultrasound application, acquiring at least one characterization image (Ic) of the humans organic tissues;
· detecting in the characterization image (Ic, the presence of a the presence of a high-level echo line (16)corresponding to agglomerations of precipitated calcium ; and
· from the contrast border (16), determining the extent of the ultrasound lesion.

2. A method according to claim 1, **characterized in that** it consists in detecting, in the characterization image (Ic), the presence of a contrast border (16) that is closed.

3. A method according to claim 1 or claim 2, **characterized in that** it consists in identifying, in the characterization image (Ic), the ultrasound lesion inside the contrast border (16).

4. A method according to any one of claims 1 to 3, **characterized in that** it consists in acquiring an ultrasound characterization image (Ic), and in identifying, within the contrast border (16) of the ultrasound characterization image (Ic), a high-level echo zone (14) and a low-level echo zone (15) located close to the high-level echo zone (14) and the contrast border (16) .

5. A method according to any one of claims 1 to 4, **characterized in that** it consists in:
· acquiring a reference image (Ir) of the organic tissues prior to applying the ultrasound, so as to visualize the tumor and compare the reference image (Ir) with the characterization image in order to monitor therapeutic margins.

6. A method according to claim 5, **characterized in that** it consists in processing the reference and characterization images (Ir, Ic) in order to determine the extent of the ultrasound lesion.

7. A method according to claim 4 or claim 5, **characterized in that** it consists in calculating a ratio between the area defined by the ultrasound lesion in the characterization image (Ic) and the area defined by the tumor in the reference image (Ir) in order to deduce a margin size for the extent of the lesion.

8. A method according to claim 5 or claim 6, **characterized in that** it consists in using a plurality of characterization images (Ic) and a plurality of reference images (Ir) to calculate a ratio between the volume defined by the ultrasound lesion in the characterization images (Ic) and the volume defined by the tumor in the reference images (Ir) in order to deduce therefrom a margin size for the extent of the ultrasound lesion.

9. A method according to claims 1 to 8, **characterized in that** it consists in acquiring the characterization image (Ic) after a period lying in the range six days to thirty days, and preferably of about eight days.

10. A method according to claims 1 to 9, **characterized in that** it consists in characterizing the lesion of organic tissues corresponding to liver tissues.
